# EUROPEAN PATENT APPLICATION

(11) **EP 4 431 529 A1**
(43) Date of publication of application: **18.09.2024**
(21) Application number: 22892037.7
(22) Date of filing: 10.11.2022
(51) Int. Cl.: C07K 19/00, C12N 15/62, C12N 15/63, C12N 5/10, A61K 38/17, A61P 17/00, A61P 27/02

(54) **ANTI-IL-17/VEGF BIFUNCTIONAL FUSION PROTEIN AND USE THEREOF**

(30) Priority: 11.11.2021 CN 202111335581
(71) Applicant: Sunshine Guojian Pharmaceutical (Shanghai) Co., Ltd., China (Shanghai) Pilot Free Trade Zone Shanghai 201203 (CN)
(72) Inventor: DENG, Lan, Shanghai 201203 (CN); HUANG, Haomin, Shanghai 201203 (CN); ZHU, Zhenping, Shanghai 201203 (CN); LIU, Lifen, Shanghai 201203 (CN); WANG, Lihua, Shanghai 201203 (CN); MENG, Yun, Shanghai 201203 (CN)
(74) Representative: Canzler & Bergmeier Patentanwälte Partnerschaft mbB
(86) International application number: PCT/CN2022/131063
(87) International publication number: WO 2023/083243

(57) **Abstract**

The present invention relates to the technical field of fusion proteins, and relates in particular to an anti-IL-17/VEGF bifunctional fusion protein and a use thereof. The anti-IL-17/VEGF bifunctional fusion protein comprises an anti-IL-17 antibody and a protein that specifically binds to VEGF The protein that specifically binds to VEGF is VEGFR, and the protein that specifically binds to VEGF is connected to the anti-IL-17 antibody directly or by means of a linker. The anti-IL-17/VEGF bifunctional fusion protein may simultaneously block IL-17 and VEGF signaling pathways, and the anti-IL-17/VEGF bifunctional fusion protein may be used to prepare drugs for treating IL-17 and/or VEGF overexpression.

## Description

### FIELD OF DISCLOSURE

The present disclosure relates to the field of fusion protein technology, in particular to an anti IL-17/VEGF bifunctional fusion protein and a use thereof.

### DESCRIPTION OF RELATED ARTS

Interleukin 17 (IL-17) is an inflammatory cytokine that is secreted mainly by helper T cells (Th17). Other T cells and innate immune cells such as mast cells and neutrophils also secrete small amounts of IL-17. The IL-17 family consists of six members (A, B, C, D, E, F, and A/F) that exist as homo- or heterodimers. IL-17 receptors contain IL-17RA, IL-17RB, IL-17RC, IL-17RD, IL-17RE, and SEF. Homodimer IL-17A, IL-17F and heterodimer IL-17A/F are the main forms present, acting together on IL-17RA and IL-17RC. IL-17A has a very low expression level in normal physiological state. However, in pathological state, IL-17A is secreted in large quantities, which binds to IL-17R on the cell surface and activates downstream signaling pathways, such as NF-kB and JNK, through signaling complex IL-17R-Act1-TRAF6, and participates in inflammatory response, playing an important role in the pathologic response of inflammation and autoimmune diseases (e.g., psoriasis, rheumatoid arthritis). Monoclonal antibody drugs against IL-17 have now become an important treatment for psoriasis.

Psoriasis is a common relapsing and inflammatory skin disease. Keratinocyte (KC) hyperproliferation, inflammatory cell infiltration, and neovascularization are the three elements of its histopathology. Current studies have shown that microvessels in the papillary layer in psoriasis lesions are dilated and tortuous, with an increased permeability and an increased number of blood vessels, occurring mainly in the post-capillary venules. The earliest pathologic change that occurs in psoriasis is alterations in the distribution and formation of blood vessels. Abnormally dilated blood vessels are also commonly seen in the non-lesional skin of patients with psoriasis. And studies have further shown that both mRNA and protein expression levels of vascular endothelial growth factor (VEGF) are significantly higher in skin lesions of patients with psoriasis than that in normal human skin. Thus, antiangiogenic drugs may be an effective way to treat psoriasis, and drugs that target both IL-17 and VEGF are likely to have additive efficacy in the treatment of psoriasis.

In addition, several studies have shown that IL-17 levels are significantly high in diabetic retinopathy, choroidal neovascularization (CNV), and macular diseases. In the laser-induced CNV model, injection of anti-IL-17 antibody, or anti-VEGF antibody, can significantly reduce neovascularization and leakage. Thus drugs that target both IL-17 and VEGF are expected to be innovative new therapies for retinopathies such as macular disease.

### SUMMARY OF THE PRESENT INVENTION

The present disclosure provides a novel anti-IL-17/VEGF bifunctional fusion protein that blocks both IL-17 and VEGF signaling pathways. The present disclosure also provides a nucleic acid molecule encoding the bifunctional fusion protein; an expression vector comprising the nucleic acid molecule; a host cell comprising the expression vector; a preparation method for the bifunctional fusion protein; a pharmaceutical composition containing the bifunctional fusion protein, a use of the bifunctional fusion protein or pharmaceutical composition in the preparation of drugs for treating diseases associated with IL-17 and/or VEGF overexpression; and a method for treating diseases in which IL-17 and/or VEGF are overexpressed using the fusion protein or the pharmaceutical composition.

The present disclosure provides the following technical solutions.

A first aspect of the present disclosure provides an anti-IL-17/VEGF bifunctional fusion protein comprising an anti-IL-17 antibody and a protein that binds specifically to VEGF, the protein that binds specifically to VEGF is VEGFR, and the protein that binds specifically to VEGF is linked to the anti-IL-17 antibody directly or via a linker.

The VEGFR can be an intact receptor protein or an extracellular domain of VEGFR.

In a preferred embodiment, the protein that binds specifically to VEGF is the extracellular domain of VEGFR. Exemplary extracellular domains of VEGFR are for example the D2 domain or the D3 domain of VEGFR1; the D2 domain, D3 domain, or D4 domain of VEGFR2; the D1 domain, D2 domain, or D3 domain of VEGFR3.

In a preferred embodiment, the protein that specifically binds to VEGF is the extracellular domain of VEGFR1.

In a preferred embodiment, the protein that specifically binds to VEGF is the D2 domain of VEGFR1.

The anti-IL-17 antibody may be any anti-IL-17 antibody as long as it inhibits or reduces the binding of IL-17 to its ligand. Preferably, the anti-IL-17 antibody is a 608 antibody.

Specifically, the heavy chain of the anti-IL-17 antibody comprises complementary determining regions HCDR1-3, wherein the amino acid sequence of HCDR1 is shown as SEQ ID NO: 14, the amino acid sequence of HCDR2 is shown as SEQ ID NO: 15, and the amino acid sequence of HCDR3 is shown as SEQ ID NO: 16; the light chain of the anti-IL-17 antibody comprises complementary determining regions LCDR1-3, wherein the amino acid sequence of LCDR1 is shown as SEQ ID NO: 17, the amino acid sequence of LCDR2 is shown as SEQ ID NO: 18, and the amino acid sequence of LCDR3 is shown as SEQ ID NO: 19.

In a preferred embodiment, the amino acid sequence of the heavy chain variable region of the anti-IL-17 antibody is shown as SEQ ID NO: 20 and the amino acid sequence of the light chain variable region of the anti-IL-17 antibody is shown as SEQ ID NO: 21.

In a preferred embodiment, the anti-IL-17 antibody is a monoclonal antibody.

In a preferred embodiment, the anti-IL-17 antibody is a humanized antibody.

In a preferred embodiment, the anti-IL-17 antibody is an IgG type antibody. Examples include the IgG1, IgG2 or IgG4 type full-length antibodies.

In a preferred embodiment, the heavy chain of the anti-IL-17 antibody has an amino acid sequence shown as SEQ ID NO: 4 and the light chain of the anti-IL-17 antibody has an amino acid sequence shown as SEQ ID NO: 1.

In other embodiments, the anti-IL-17 antibody may also be other antibodies that compete with the 608 antibody for binding IL-17.

In some specific embodiments of the present disclosure, the anti-IL-17 antibody may also be obtained by substituting, deleting, or adding one or more (specifically 1-50, 1-30, 1-20, 1-10, 1-5, or 1-3) amino acids in the heavy chain amino acid sequence shown as SEQ ID NO: 4 or in the light chain amino acid sequence shown as SEQ ID NO: 1. The amino acid sequence after substitution, deletion, or addition can have more than 80%, 85%, 90%, 93%, 95%, 97%, or 99% identity to SEQ ID NO: 4 or SEQ ID NO: 1.

In one embodiment, the D2 domain of VEGFR can be wild-type or mutant.

The amino acid sequence of wild-type VEGFR-D2 is shown as SEQ ID NO: 34.

The mutant of the D2 domain of VEGFR is obtained by substituting, deleting, or adding one or more (specifically 1-50, 1-30, 1-20, 1-10, 1-5, or 1-3) amino acids in the amino acid sequence shown as SEQ ID NO: 34. The amino acid sequence after substitution, deletion, or addition can have more than 80%, 90%, 90%, 93%, 95%, 97%, or 99% identity to SEQ ID NO: 34.

In a preferred embodiment, the mutant of VEGFR-D2 has a mutation at position 138 of the amino acid sequence shown as SEQ ID NO: 34.

Further, the mutant of VEGFR-D2 has a substitution mutation at position 138 of the amino acid sequence shown as SEQ ID NO: 34.

Further, the mutant of VEGFR-D2 is selected from 138M/A, 138M/P, and 138M/T. The three mutations have unexpected technical effects, significantly reducing heavy chain breaks in the fusion protein and improving accelerated thermal stability.

In a preferred embodiment, the linker may be a flexible polypeptide sequence, such as (G4S)x, wherein x may be 1, 2, 3, 4, 5, or 6.

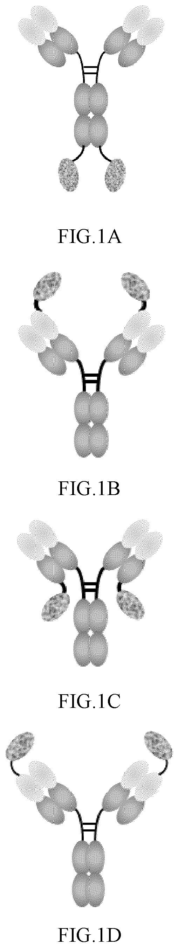

In certain preferred embodiments, the C-terminal (as shown in FIG. 1A) or N-terminal (as shown in FIG. 1B) end of the heavy chain of the anti-IL-17 antibody IgG is tandemly coupled with the protein that binds specifically to VEGF to form a heavy chain of the bifunctional fusion protein.

In certain preferred embodiments, the C-terminal end (shown in FIG. 1C) or the N-terminal end (shown in FIG. 1D) of the light chain of the anti-IL-17 antibody IgG and the protein that binds specifically to VEGF are tandemly linked to form a light chain of the bifunctional fusion protein.

In a preferred embodiment, the C-terminal or N-terminal end of the heavy chain of the anti-IL-17 antibody is linked to the D2 domain of VEGFR1. In another preferred embodiment, the C- terminal or N- terminal end of the light chain of the anti-IL-17 antibody is linked to the D2 domain of VEGFR1.

To reduce the breakage of the fusion protein, the terminal amino acid of the Fc can be mutated, such as mutating the amino acid K at the end of the human IgG1 type Fc segment in the embodiment to A.

In a preferred embodiment, the bifunctional fusion protein has a heavy chain amino acid sequence shown as SEQ ID NO: 2, SEQ ID NO: 3, SEQ ID NO: 4, SEQ ID NO: 7, SEQ ID NO: 8, SEQ ID NO: 9, or SEQ ID NO: 10, and the light chain amino acid sequence of the bifunctional fusion protein is shown as SEQ ID NO: 1, SEQ ID NO: 5, or SEQ ID NO: 6.

The second aspect of the present disclosure provides a nucleic acid molecule, which encodes the fusion protein.

In a preferred embodiment, the nucleic acid sequence of the nucleic acid molecule encoding the heavy chain of the fusion protein is shown as SEQ ID NO: 25, SEQ ID NO: 26, SEQ ID NO: 27, SEQ ID NO: 30, SEQ ID NO: 31, SEQ ID NO: 32, or SEQ ID NO: 33, and the nucleic acid sequence of the nucleic acid molecule encoding the light chain of the fusion protein is shown as SEQ ID NO: 24, SEQ ID NO: 28, or SEQ ID NO: 29.

It is known to those of skill in the art that the nucleic acid molecule encoding the amino acid sequence of the above-described fusion protein may be suitably introduced with substitutions, deletions, alterations, insertions, or additions to provide a homologue of the nucleic acid molecule.

The third aspect of the present disclosure provides an expression vector containing the aforementioned nucleic acid molecule.

The fourth aspect of the present disclosure provides a host cell comprising the aforementioned expression vector.

The fifth aspect of the present disclosure provides a preparation method for the bifunctional fusion protein comprising the following steps:
a) culturing a host cell under an expression condition to express the anti-IL-17/VEGF bifunctional fusion protein;
b) isolating and purifying the bifunctional fusion protein in step a).

The sixth aspect of the present disclosure provides a pharmaceutical composition comprising an effective amount of the aforementioned bifunctional fusion protein and one or more pharmaceutically acceptable carriers or excipients.

The seventh aspect of the present disclosure provides a use of the aforementioned bifunctional fusion protein and pharmaceutical composition in the preparation of drugs for treating diseases associated with IL-17 and/or VEGF overexpression.

According to the present disclosure, the disease in which IL-17 and/or VEGF is overexpressed is selected from inflammatory skin diseases and ophthalmic diseases.

The inflammatory skin disease is, for example, psoriasis.

The ophthalmic disease is, for example, diabetic retinopathy, choroidal neovascularization (CNV), or macular disease.

The macular disease is, for example, wet age-related macular degeneration.

The eighth aspect of the present disclosure provides a use of the above-mentioned bifunctional fusion protein and pharmaceutical composition in the preparation of drugs for treating inflammatory skin diseases or ophthalmic diseases.

The inflammatory skin disease is, for example, psoriasis.

The ophthalmic disease is, for example, diabetic retinopathy, choroidal neovascularization (CNV), or macular disease.

The macular disease is, for example, wet age-related macular degeneration.

When the anti-IL-17/VEGF bifunctional fusion protein and its pharmaceutical compositions in the present disclosure are administered to animals including humans, the dosage administered varies depending on the age and weight of the patient, the characteristics and severity of the disease, and the route of administration, and may be referred to the results of the animal experiments and various circumstances. The total amount of the dosage administered should not exceed a certain range. On the basis of common knowledge in the field, each of the above preferred conditions can be arbitrarily combined to obtain each preferred example of the present disclosure.

A ninth aspect of the present disclosure provides a method for treating diseases associated with IL-17 and/or VEGF overexpression comprising administering to a subject in need thereof the bifunctional fusion protein or pharmaceutical composition as described above.

According to the present disclosure, the disease in which IL-17 and/or VEGF is overexpressed is selected from inflammatory skin diseases and ophthalmic diseases.

The inflammatory skin disease is, for example, psoriasis.

The ophthalmic disease is, for example, diabetic retinopathy, choroidal neovascularization (CNV), or macular disease.

The macular disease is, for example, wet age-related macular degeneration.

A tenth aspect of the present disclosure provides a method for treating inflammatory skin diseases or ophthalmic diseases comprising administering to a subject in need thereof the bifunctional fusion protein or pharmaceutical composition as described above.

The inflammatory skin disease is, for example, psoriasis.

The ophthalmic disease is, for example, diabetic retinopathy, choroidal neovascularization (CNV), or macular disease.

The macular disease is, for example, wet age-related macular degeneration.

The anti-IL-17/VEGF bifunctional fusion protein and pharmaceutical composition thereof need to be administered in a therapeutically effective amount when being administered to a subject. The therapeutically effective amount is an amount that is effective in the treatment of a disease in which IL-17 and/or VEGF is overexpressed. Specifically, the anti-IL-17/VEGF bifunctional fusion protein and pharmaceutical composition thereof, when being administered to a subject, are administered in doses that vary depending on the age and weight of the patient, the characteristics and severity of the disease, and the route of administration, which may be referred to the results of animal experiments and various circumstances. The total amount administered should not exceed a certain range.

An eleventh aspect of the present disclosure provides a fusion protein comprising the D2 domain of VEGFR1, the D2 domain of VEGFR1 is mutated. The fusion protein has an improved stability.

In a preferred embodiment, the fusion protein further comprises at least one heterologous domain, the heterologous domain comprises, but not limited to, an antibody, an Fc fragment, to improve targeting, stability, non-toxicity, tumor killing activity, etc.

In a preferred embodiment, the D2 domain of VEGFR1 is mutated at position 138 of the amino acid sequence shown as SEQ ID NO: 34.

Still further, the D2 domain of VEGFR1 has a substitution mutation at position 138 of the amino acid sequence shown as SEQ ID NO: 34.

Still further, the D2 domain of VEGFRlhas a138M/A, 138M/P, or 138M/T mutation.

The positive effects of the present disclosure include that the bifunctional fusion protein of the present disclosure binds IL-17 and VEGF with high affinity, and its affinity for IL-17 is comparable to that of the anti-IL-17 monoclonal antibody positive control 608. The fusion protein of the present disclosure can effectively block the binding of IL-17 receptor to IL-17, and its blocking ability is comparable to the anti-IL-17 monoclonal antibody positive control 608. The fusion protein of the present disclosure can effectively block the interaction between VEGF and its receptor KDR, and its blocking ability is superior to the anti-VEGF monoclonal antibody positive control Bevacizumab. The fusion protein of the present disclosure can effectively inhibit IL-17-induced secretion of IL-6 as well as VEGF in cellular level pharmacodynamic assays. Effective treatment of psoriasis as well as choroidal neovascularization can be realized in pharmacodynamic experiments at the animal level. In summary, the fusion protein of the present disclosure have the potential to treat diseases associated with IL-17 and VEGF activity.

### BRIEF DESCRIPTION OF THE DRAWINGS

FIGs. 1A to 1D show schematic structures of different bifunctional fusion proteins of the present disclosure, wherein the light gray portion represents the variable region, the dark gray portion represents the constant region, and the texture filled portion represents the D2 domain of VEGFR1.
FIG. 2A to FIG. 2H show graphs indicating the purity results of the anti-IL-17/VEGF bifunctional fusion proteins in Example 2 of the present disclosure as detected by HPLC, and FIG. 2A to FIG. 2H sequentially show the results of 17V01, 17V02, 17V03, 17V04, 17V05, 17V06, 17V07, and 17V08.
FIG. 3A shows the ELISA assay results of the affinity of the anti-IL-17/VEGF bifunctional fusion protein to IL-17 in Example 3 of the present disclosure.
FIG. 3B shows the ELISA assay results of the affinity of the anti-IL-17/VEGF bifunctional fusion protein to VGEF in Example 3 of the present disclosure.
FIG. 4 shows the results of inhibition of IL-6 secretion induced by IL-17A- in HeLa cells by the anti-IL-17/VEGF bifunctional fusion protein in Example 6 of the present disclosure.
FIG. 5 shows the results of inhibition of the binding of VEGF to its receptor KDR by the anti- IL-17/VEGF bifunctional fusion protein in Example 7 of the present disclosure.
FIG. 6 shows the rate of change in vascular leakage area in animals after administration of the anti-IL-17/VEGF bifunctional fusion protein in Example 10 of the present disclosure.
FIG. 7 shows statistical data for 3-4 grade leakage spots in Example 10 of the present disclosure.
FIG. 8 shows the results of IB4 immunofluorescence staining after choroidal flatmounting in Example 10 of the present disclosure.

### DETAILED DESCRIPTION OF THE EMBODIMENTS

The following examples are further illustration of the present disclosure and should not be construed as a limitation of the present disclosure. Embodiments do not include a detailed description of conventional methods or methods conventional in the art, such as methods for preparing nucleic acid molecules, methods for constructing vectors and plasmids, methods for inserting genes encoding proteins into such vectors and plasmids or methods for introducing plasmids into host cells, methods for culturing host cells, and so on. Such methods are well known to those of ordinary skill in the art and are described in a number of publications, including Sambrook, J., Fritsch, E.F. and Maniais, T. (1989) Molecular Cloning: A Laboratory Manual, 2nd edition, Cold spring Harbor Laboratory Press.

In the present disclosure, the term "bifunctional fusion protein" refers to a new polypeptide sequence obtained by fusing two or more identical or different polypeptide sequences. The term "fusion" refers to a direct linkage by a peptide bond or an effective linkage by means of one or more connecting peptides (peptide linkers). The term "connecting peptide (linker)" refers to a short peptide that connects two polypeptide sequences, typically the peptides of 2-30 amino acids in length.

In the present disclosure, the term "antibody (abbreviated as Ab)" includes full-length antibodies and antibody fragments. "Full-length antibody" refers to an isotetrameric glycoprotein of approximately 150,000 daltons, consisting of two identical light chains (LC) and two identical heavy chains (HC). Each heavy chain has a variable region (VH) at one end, followed by a constant region. The constant region of the heavy chain consists of three domains CH1, CH2, and CH3. Each light chain has a variable region (VL) at one end and a constant region at the other end, and the constant region of the light chain includes a domain CL. The constant region of the light chain pairs with the CH1 domain of the constant region of the heavy chain, and the variable region of the light chain pairs with the variable region of the heavy chain. Constant regions are not directly involved in antibody-antigen binding, but they exhibit different effector functions, such as antibody-dependent cell-mediated cytotoxicity (ADCC), etc. The heavy chain constant region includes subtypes of IgG1, IgG2, IgG3, and IgG4; The light chain constant regions include κ (Kappa) or λ (Lambda). The heavy and light chains of the antibody are covalently linked together by a disulfide bond between the CH1 domain of the heavy chain and the CL domain of the light chain, and the two heavy chains of the antibody are covalently linked together by an inter-polypeptide disulfide bond formed between the hinge regions. "Antibody fragment" means a functional fragment which is derived from an antibody and retains the ability to bind to an antigenic epitope, including, but not limited to, scFv, Fv, Fd, Fab, F(ab')2, and F(ab').

In the present disclosure, the term "monoclonal antibody" refers to an antibody obtained from a substantially homogeneous group, i.e., the group mainly contains individual antibodies that are identical and a small number of naturally occurring mutations. Monoclonal antibodies are highly specific to a single antigenic site. Moreover, unlike conventional polyclonal antibody preparations, which are usually mixtures of different antibodies having different antigenic determinants, each monoclonal antibody has a single determinant. In addition to their specificity, monoclonal antibodies have the advantage that they can be synthesized by hybridoma culture and will not be contaminated by other immunoglobulins.

In the present disclosure, the term "humanized" means that the CDR is derived from an antibody of a non-human species (preferably mouse), and that the residual portion of the antibody molecule, including the framework region and the constant region, is derived from the human antibody. In addition, the frame region residues can be altered to maintain binding affinity.

In the present disclosure, the terms "anti" and "binding" refer to a non-random binding reaction between two molecules, such as a reaction between the antibody and the antigen against which it is directed. Typically, the antibody binds the antigen with an equilibrium dissociation constant (KD) less than about 10-7M, e.g., less than about 10-8M, 10-9M, 10-10M, 10-11M, etc. The term "KD" refers to the equilibrium dissociation constant for a given antibody-antigen interaction, which is used to describe the binding affinity between the antibody and antigen. The smaller the equilibrium dissociation constant, the tighter the antibody-antigen binding and the higher the affinity between antibody and antigen. For example, the binding affinity of an antibody to an antigen is determined in a BIACORE instrument using Surface Plasmon Resonance (abbreviated as SPR) or the relative binding affinity of an antibody to an antigen is determined using ELISA.

In the present disclosure, the term "D2 domain of VEGFR1" refers to the D2 domain of the extracellular domain of VEGFR1, including the natural sequence and variants thereof (comprising at least 1 amino acid mutation). The mutated domain retains binding activity to VEGF.

In the present disclosure, the term "expression vector" means a vector, such as a plasmid, a viral vector (e.g., an adenovirus, a retrovirus), a phage, a yeast plasmid, etc,, which carries an expression cassette for expression of a specific target protein or other substance. Examples include conventional expression vectors of the art comprising appropriate regulatory sequences, such as promoters, terminators, enhancers, etc., viral vectors (e.g., adenoviruses, retroviruses), plasmids, phages, yeast plasmids, and other vectors. The expression vectors preferably include pDR1, pcDNA3.4(+), pDHFR, or pTT5. Once the required sequence is obtained, recombination can be used to obtain a large quantity of this sequence. This usually involves cloning the sequence into a vector, transferring the vector into cells, and then isolating the sequence from the proliferated host cell by conventional methods.

In the present disclosure, the term "host cell" is used to refer to a variety of host cells conventional in the art, as long as the vector can be stabilized for self-replication and the nucleic acid molecules it carries can be effectively expressed. The host cells comprise prokaryotic-expressing cells and eukaryotic-expressing cells, wherein the host cells are preferably selected from: COS, CHO, NS0, sf9, sf21, DH5α, BL21 (DE3), TG1, BL21 (DE3), 293F cells, and 293E cells.

In the present disclosure, the term "pharmaceutical composition" contains the bifunctional fusion protein described above and a pharmaceutically acceptable carrier or excipient. Typically, these substances may be formulated in a non-toxic, inert, and pharmaceutically acceptable aqueous carrier medium, wherein the pH is typically about 4-8, and preferably the pH is about 5-7. The pH may vary depending on the nature of the substance being formulated and the condition to be treated. The formulated pharmaceutical composition can be administered by conventional routes which include (but are not limited to): intravenous injection, subcutaneous injection, local injection, intramuscular injection, intratumoral injection, intraperitoneal injection, intracranial injection, and intracavitary injection.

In the present disclosure, the term "pharmaceutical composition" refers to pharmaceutical formulations that provide stable therapeutic efficacy by ensuring the conformational integrity of the core amino acid sequences of the bifunctional fusion protein disclosed herein while also protecting the multifunctional groups of the protein from degradation (including, but not limited to, coagulation, deamination, and oxidation).

"Pharmaceutically acceptable" means that when drugs are properly administered to animals or humans, they do not produce adverse, allergic, or other side effects.

"Pharmaceutically acceptable carrier or excipient" should be compatible with the active ingredient, i.e., capable of being mixed with the active ingredient without substantially reducing the effect of the drug under normal conditions. Specific examples of some substances that may be used as pharmaceutically acceptable carriers or excipients are sugars, such as lactose, glucose, and sucrose; starch, such as corn starch and potato starch; cellulose and its derivatives, such as sodium methyl cellulose, ethyl cellulose, and methyl cellulose; Gummi Tragacanthae powder; malt; gelatin; talc; solid lubricants such as stearic acid and magnesium stearate; calcium sulfate; vegetable oils, such as peanut oil, cottonseed oil, sesame oil, olive oil, corn oil, and cocoa butter; polyols, such as propylene glycol, glycerol, sorbitol, mannitol, and polyethylene glycol; alginic acid; emulsifiers, such as Tween; wetting agents, such as sodium lauryl sulfate; colorants; flavorings; tabletting agents, stabilizers; diluents; excipients; antioxidants; preservatives; pyrogen free water; isotonic salt solution; and buffers, such as phosphate buffers. The above substances are used as needed to help the stability of the formulation, improve the activity or bioavailability of the formulation, or produce an acceptable mouthfeeling or odor in the case of oral administration.

The pharmaceutical composition of the present disclosure contain a safe and effective amount (e.g., 0.001-99wt%, preferably 0.01-90wt%, more preferably 0.1-80wt%) of the bifunctional fusion protein of the present disclosure described above, as well as the pharmaceutically acceptable carrier. Such carrier includes (but is not limited to): saline, buffer, glucose, water, glycerol, ethanol, and combinations thereof. Pharmaceutical preparation should be matched to the mode of administration. The pharmaceutical composition of the present disclosure may be made in the form of injections, for example, prepared by conventional methods using saline or an aqueous solution containing glucose and other excipients. Pharmaceutical compositions in the form of such as injections and solutions should be manufactured under sterile conditions. The active ingredient is administered in a therapeutically effective amount, e.g., about 10-50 micrograms/kg body weight per day. In addition, the bifunctional fusion protein of the present disclosure can also be used in combination with other therapeutic agents.

In the present disclosure, the term "effective amount" refers to the amount or dose of the pharmaceutical composition of the present disclosure that, when being administered to a subject, produces a desired effect in the treated individual, wherein the desired effect includes an improvement in the individual's condition. The term "subject" includes, but is not limited to, mammals such as humans, non-human primates, rats, and mice.

In the method of the present disclosure for treating IL-17 and/or VEGF overexpression diseases, the "treating" or "therapy" comprises preventing or alleviating a state, delaying the rate at which a state occurs or slowing the rate at which a state develops, reducing the risk of developing a state, preventing or delaying the development of symptoms associated with a state, reducing or terminating symptoms associated with a state, producing a complete or partial reversal of a state, curing a state, or a combination of the foregoing.

In some embodiments, the methods described herein may be further administered in combination with other compounds or other therapeutic regimens in the prior art. The sequence numbers corresponding to the sequences involved in the following embodiments are summarized in the table below.

### SEQ ID NO: Sequence description and specific sequences

| SEQ ID NO: | Sequence description and specific sequences |
|---|---|
| 1 | Light chain amino acid sequence of 17V01, 17V02, 17V05, 17V06, 17V07, 17V08, and 608 monoclonal antibody |
| 2 | Heavy chain amino acid sequence of 17V01 |
| 3 | Heavy chain amino acid sequence of 17V02 |
| 4 | Heavy chain amino acid sequence of 17V03, 17V04, and 608 monoclonal antibody |
| 5 | Light chain amino acid sequence of 17V03 |
| 6 | Light chain amino acid sequence of 17V04 |
| 7 | Heavy chain amino acid sequence of 17V05 |
| 8 | Heavy chain amino acid sequence of 17V06 |
| 9 | Heavy chain amino acid sequence of 17V07 |
| 10 | Heavy chain amino acid sequence of 17V08 |
| 11 | FC-D2 amino acid sequence |
| 12 | Heavy chain amino acid sequence of Bevacizumab |
| 13 | Light chain amino acid sequence of Bevacizumab |
| 14 | Amino acid sequence of 608 monoclonal antibody-HCDR1 |
| 15 | Amino acid sequence of 608 monoclonal antibody-HCDR2 |
| 16 | Amino acid sequence of 608 monoclonal antibody-HCDR3 |
| 17 | Amino acid sequence of 608 monoclonal antibody-LCDR1 |
| 18 | Amino acid sequence of 608 monoclonal antibody-LCDR2 |
| 19 | Amino acid sequence of 608 monoclonal antibody-LCDR3 |
| 20 | Amino acid sequence of the heavy chain variable region of the 608 monoclonal antibody |
| 21 | Amino acid sequence of the light chain variable region of the 608 monoclonal antibody |
| 22 | Amino acid sequence of the constant region of human IgG1 |
| 23 | Amino acid sequence of the constant region of the human kappa |
| 24 | Light chain nucleic acid sequence of 17V01, 17V02, 17V05, 17V06, 17V07, 17V08, and 608 monoclonal antibody |
| 25 | Heavy chain nucleic acid sequence of 17V01 |
| 26 | Heavy chain nucleic acid sequence of 17V02 |
| 27 | Heavy chain nucleic acid sequence of 17V03, 17V04, and 608 monoclonal antibody |
| 28 | Light chain nucleic acid sequence of 17V03 |
| 29 | Light chain nucleic acid sequence of 17V04 |
| 30 | Heavy chain nucleic acid sequence of 17V05 |
| 31 | Heavy chain nucleic acid sequence of 17V06 |
| 32 | Heavy chain nucleic acid sequence of 17V07 |
| 33 | Heavy chain nucleic acid sequence of 17V08 |
| 34 | Amino acid sequence of the D2 domain of VEGFR1 |

### Embodiment 1: Preparation of anti-IL-17/VEGF bifunctional fusion proteins

The present disclosure constructs an antibody fusion protein by tandemly linking the D2 domain of VEGFR1 to the C-terminal (as shown in Figure 1A) or N-terminal (as shown in Figure 1B) of the IgG heavy chain of the anti-IL-17 monoclonal antibody (608 monoclonal antibody), as well as to the C-terminal (as shown in Figure 1C) or N-terminal (as shown in Figure 1D) of the light chain of the anti-IL-17 monoclonal antibody (608 monoclonal antibody). The 608 monoclonal antibody was derived from the humanized anti-human IL-17A monoclonal antibody in CN 201780033670.5. The D2 domain of VEGFR1 (amino acids 129th to 227th) includes the wild-type D2 domain and the D2 domain that has undergone specific mutation, and the structural description of each fusion protein is shown in Table 1.

**Table 1 Structural description of fusion proteins**

| Fusion Protein | D2 Domain | Linker | Connection Method |
|---|---|---|---|
| 17V01 | wild-type | (G4S) 2 | The D2 domain is attached to the C-terminal end of the IgG heavy chain of the anti-IL-17 monoclonal antibody (608 monoclonal antibody) (Figure 1A) |
| 17V02 | wild-type | (G4S) 4 | The D2 domain is attached to the N- terminal of the IgG heavy chain of the anti-IL-17 monoclonal antibody (608 monoclonal antibody) (Figure 1B) |
| 17V03 | wild-type | (G4S) 2 | The D2 domain is attached to the C-terminal end of the light chain of the anti-IL-17 monoclonal antibody (608 monoclonal antibody) (Figure 1C). |
| 17V04 | wild-type | (G4S) 4 | The D2 domain is attached to the N-terminal end of the light chain of the anti-IL-17 monoclonal antibody (608 monoclonal antibody) (Figure 1D). |
| 17V05 | 138M/A | (G4S) 2 | The D2 domain is attached to the C-terminal end of the IgG heavy chain of the anti-IL-17 monoclonal antibody (608 monoclonal antibody) (Figure 1A) |
| 17V06 | 138M/P | (G4S) 2 | The D2 domain is attached to the C-terminal end of the IgG heavy chain of the anti-IL-17 monoclonal antibody (608 monoclonal antibody) (Figure 1A) |
| 17V07 | 138M/T | (G4S) 2 | The D2 domain is attached to the C-terminal end of the IgG heavy chain of the anti-IL-17 monoclonal antibody (608 monoclonal antibody) (Figure 1A) |
| 17V08 | 138M/A; 139M/A | (G4S) 2 | The D2 domain is attached to the C-terminal end of the IgG heavy chain of the anti-IL-17 monoclonal antibody (608 monoclonal antibody) (Figure 1A) |

### Embodiment 2 Preparation of anti-IL-17/VEGF bifunctional fusion proteins

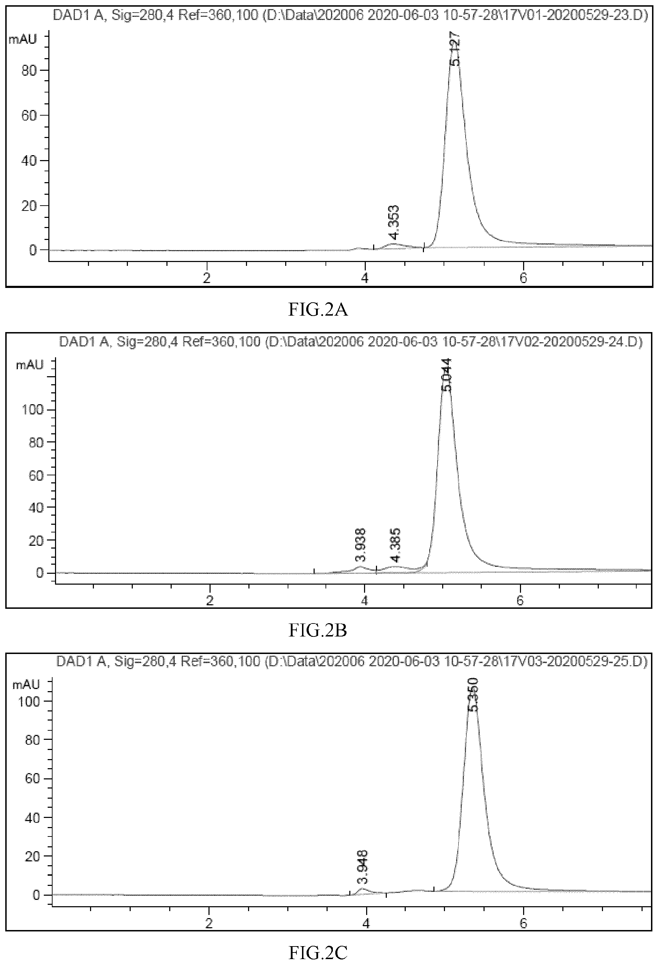

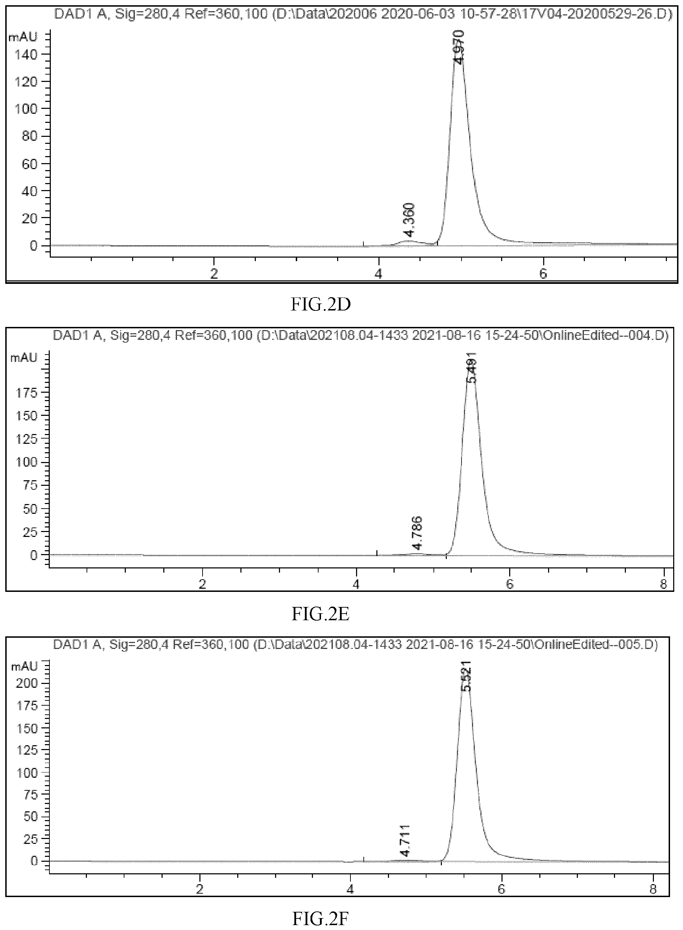

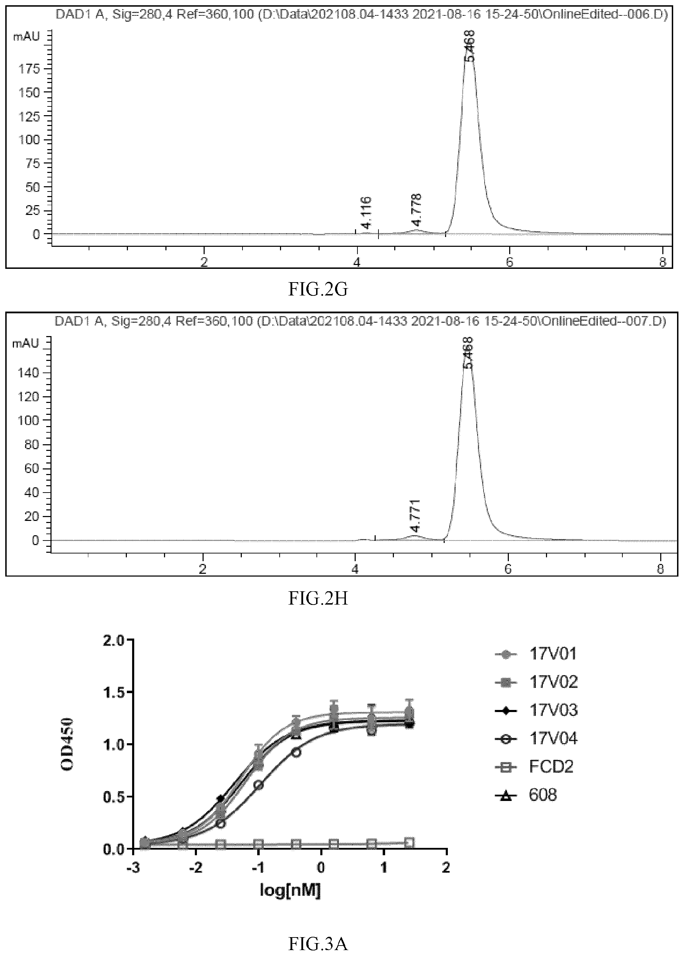

The DNA fragments of the heavy and light chains of the anti-IL-17/VEGF bifunctional fusion protein were subcloned into the pcDNA3.4 vector (purchased from thermofisher, A14697), respectively, and the recombinant plasmid was extracted and co-transfected into CHO cells and/or 293F cells. After 7 days of cell culture, the culture solution was subjected to high-speed centrifugation and vacuum filtration through a microporous membrane, and then loaded onto a HiTrap MabSelect SuRe column. The proteins were eluted in one step with an eluent of 100 mM citrate, pH 3.5, and the target samples were recovered and dialyzed into PBS. The purified proteins were detected by HPLC. The results were shown in Figure 2A to Figure 2H, and the assay results indicate that the molecular state of the fusion proteins is homogeneous, and the purity of the monomers after a single purification ranges from 94.0% ~ 98.9%.

### Embodiment 3. Determination of affinity of anti IL-17/VEGF bifunctional fusion proteins for antigen using enzyme linked immunosorbent assay (ELISA)

### 3.1 Determination of affinity of anti-IL-17/VEGF bifunctional fusion proteins for IL-17A using ELISA

The IL-17A protein (purchased from acrobiosystems, Cat.# ILA-H5118) was coated on the plate at 100 ng/well and cultured overnight at 4°C. The plate was washed 3 times with PBST, 200 µl/well of blocking solution was added, followed by incubation 1 hour at 37°C. The plate was washed 1 time with PBST for use. The antibody was diluted to 25 nM with diluent, and samples with 8 concentration gradients were prepared using 4-fold dilution. The samples were sequentially added to the blocked ELISA plate at 100 µl/well, and incubated at 37°C for 1 hour. The plate was washed 3 times with PBST, and HRP-labeled goat anti-human Fab antibody (purchased from abcam, Cat.#ab87422) was added, followed by incubation at 37°C for 30 minutes. After washing the plate 3 times with PBST, the residual droplets were removed as much as possible on absorbent paper, 100 µl of TMB was added to each well, and the plate was placed at room temperature (20±5°C) for 5 minutes away from light. The substrate reaction was terminated by adding termination solution to each well. The OD values were read at 450 nm using the microplate reader, and GraphPad Prism6 was used to analyze the data, graphs were plotted and the EC50 was calculated.

As shown in Figure 3A, the anti-IL-17/VEGF bifunctional fusion proteins 17V01, 17V02, 17V03, 17V04 and the positive control 608 monoclonal antibody are all able to bind IL-17A effectively, with EC50 (nM) values of 0.052, 0.062, 0.041, 0.105, and 0.052, respectively, indicating comparable affinities.

### 3.2 Determination affinity of anti-IL-17/VEGF bifunctional fusion proteins for VEGF using ELISA

To test the binding ability of anti-IL-17/VEGF bifunctional fusion protein to VEGF, VEGF protein (purchased from acrobiosystems, Cat.# VE5-H4210) was coated on the plate at 100 ng/well and incubated at 4°C overnight. The plate was washed 3 times with PBST, 200 µl/well of blocking solution was added. After incubation 1 hour at 37°C, the plate was washed 1 time with PBST for use. The antibody was diluted to 25 nM with diluent, and the samples with 8 concentration gradients were prepared using 4-fold dilution. The samples were sequentially added to the blocked ELISA plate at 100 µl/well, and incubated at 37°C for 1 hour. The plate was washed 3 times with PBST, and HRP-labeled goat anti-human Fab antibody (purchased from abcam, Cat.#ab87422) was added, followed by incubation at 37°C for 30 minutes. After washing the plate 3 times with PBST, the residual droplets were removed dry as much as possible on absorbent paper, 100 µl of TMB was added to each well, and the plate was placed at room temperature (20±5°C) for 5 minutes away from light. The substrate reaction was terminated by adding termination solution to each well. The OD values were read at 450 nm using the microplate reader, and GraphPad Prism6 was used to analyze the data, graphs were plotted and the EC50 was calculated.

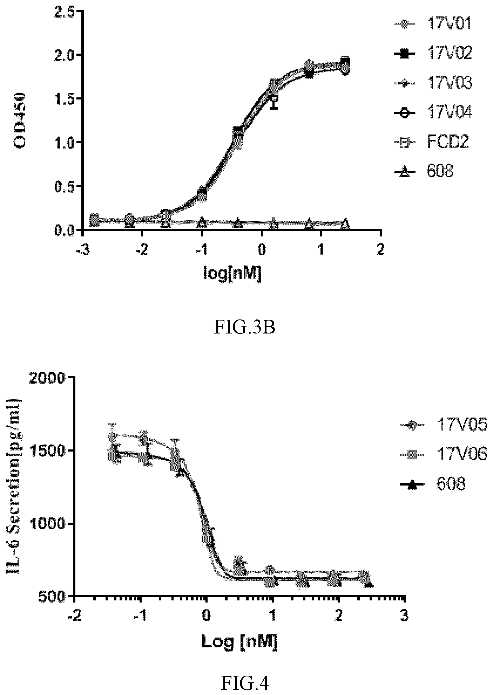

As shown in Figure 3B, the anti-IL-17/VEGF bifunctional fusion proteins 17V01, 17V02, 17V03, 17V04, and FC-D2 are all able to bind VEGF efficiently, with EC50 (nM) values of 0.379, 0.327, 0.326, 0.371, and 0.397, respectively, indicating comparable affinities. Anti-IL-17 monoclonal antibody 608 can not bind VEGF efficiently.

### Embodiment 4. Determination of the affinity dissociation constant KD of anti-IL-17/VEGF bifunctional fusion proteins against the antigen IL-17A

Kinetic parameters of the binding and dissociation between anti-IL-17/VEGF bifunctional fusion proteins and antigen IL-17A were determined using the capture method with the Biacore 8K Molecular Interaction Analyzer. HBS-EP+ buffer pH 7.4 (Cat.#BR-1007-69, GE Healthcare) was used as the dilution buffer, and 17V01, 17V05, 17V06, and 608 were diluted to 1.5 µg/ml, respectively. Six concentration gradients were set up with the highest concentration of IL-17A at 12.5 nM, and an additional concentration of 0 was set up. Antibodies were captured using microchips coupled with Protein A (Cat.#29-1275-56, GE Healthcare). IL-17A was injected as the analyte to obtain the binding-dissociation curve, and 6 M Guanidine hydrochloride (Cat.#30095516, purchased from Sinopharm Chemical Reagent Co., Ltd.) was used as the regeneration buffer, and then the process was repeated for a next cycle after elution. The data were analyzed using Biacore 8K Evaluation Software.

As shown in Table 2, the affinity of anti-IL-17/VEGF bifunctional fusion proteins 17V01, 17V05, and 17V06 for IL-17A is comparable to that of the positive control anti-IL-17 monoclonal antibody 608.

**Table 2 Affinity dissociation constant**

| stationary phase | mobile phase | ka (1/Ms) | kd (1/s) | KD (M) |
|---|---|---|---|---|
| 608 | IL-17A | 2.44E+06 | 4.00E-05 | 1.64E-11 |
| 17V01 | IL-17A | 2.55E+06 | 4.72E-05 | 1.85E-11 |
| 17V05 | IL-17A | 2.72E+06 | 5.00E-05 | 1.84E-11 |
| 17V06 | IL-17A | 2.28E+06 | 5.13E-05 | 2.25E-11 |

| | | | | |
|---|---|---|---|---|
| [00111] Note: KD is the affinity constant, ka is the binding rate constant, kd is the dissociation rate constant | | | | |

### Embodiment 5. Determination of the affinity dissociation constant KD of anti-IL-17/VEGF bifunctional fusion proteins against the antigen VEGF

Kinetic parameters of the binding and dissociation between anti-IL-17/VEGF bifunctional fusion proteins and antigenic VEGF were determined using the capture method with the Biacore 8K Molecular Interaction Analyzer. HBS-EP+ buffer pH 7.4 was used as the dilution buffer, and Bevacizumab, 17V01, 17V05, and 17V06 were diluted to 5 µg/ml, respectively. Six concentration gradients were set up with the highest concentration of VEGF at 10 nM, and an additional concentration of 0 was set up. Antibodies were captured using a microchip coupled to Protein A. VEGF was then injected as then analyte to obtain the binding-dissociation curve, and the process was repeated for a next cycle after elution with 6 M Guanidine hydrochloride as regeneration buffer. The data were analyzed using Biacore 8K Evaluation Software.

**As** shown in Table 3, the affinity of anti-IL-17/VEGF bifunctional fusion proteins 17V01, 17V05, and 17V06 for VEGF is slightly superior to that of the positive control Bevacizumab.

**Table 3: Affinity dissociation constant**

| stationary phase | mobile phase | ka (1/Ms) | kd (1/s) | KD (M) |
|---|---|---|---|---|
| Bevacizumab | VEGF165 | 1.27E+06 | 6.03E-05 | 4.74E-11 |
| 17V01 | VEGF165 | 4.08E+07 | 7.86E-04 | 1.93E-11 |
| 17V05 | VEGF165 | 3.94E+07 | 4.32E-04 | 1.10E-11 |
| 17V06 | VEGF165 | 3.79E+07 | 5.82E-04 | 1.53E-11 |

| | | | | |
|---|---|---|---|---|
| Note: KD is the affinity constant, ka is the binding rate constant, kd is the dissociation rate constant | | | | |

### Embodiment 6. Blockade effect of anti-IL-17/VEGF bifunctional fusion protein on IL-17A-induced IL-6 secretion in HeLa cells

IL-17A stimulates IL-6 secretion from epithelial cells, and stimulation of the human cervical cancer cell line HeLa using 10 ng/ml of human IL-17A results in significant human IL-6 secretion using ELISA, and the blockade effect of anti-IL-17/VEGF bifunctional fusion proteins on human IL-6 secretion levels from HeLa induced by human IL-17A can be detected using this system. HeLa cells cultured to the exponential growth phase were discarded of the medium, digested with trypsin, centrifuged at 300 g for 5 min, resuspended, and counted. The cells with adjusted density were inoculated into a 96-well plate at 10,000 cells/well, with 100 µl per well. The plate was placed in a cell culture incubator for 2 hours. Each sample to be tested was diluted with medium to a starting concentration of 40 µg/ml, and serially diluted 3-fold across 10 concentrations, 2 replicate wells were set up for each group, and 70 µl was transferred to a new 96-well plate. The concentration of IL-17A was diluted to 40 ng/ml with culture medium and 70 µl was added to the 96-well plate containing 70 µl of the diluted antibody. The solution was added to the inoculated HeLa cells at 100 µl per well. The final concentration of antibody was 10 µg/ml and the final concentration of IL-17A was 10 ng/ml. The plate was incubated overnight in a 37 °C incubator.

The rat anti-human IL-6 antibody was diluted to 2.5 µg/ml with coating solution, added to the ELISA plate at 100 µl/well, and incubated at 4°C overnight. The plate was washed 3 times with PBST, 200 µl/well of blocking solution was added, and the plate was incubated at 37°C for 2 hours and then washed 3 times with PBST for use. Preparation of IL-6 standard: IL-6 standard products was diluted with culture medium so that the initial concentration was 50 ng/ml, sequentially followed by 2-fold serial dilutions to obtain a total of 12 gradients. Each gradient was added to the well-coated ELISA plate at 100 µl per well, and 2 duplicate wells were set up for each concentration. The overnight incubated HeLa cells were mixed, and 100 µl of the mixture was diluted 2-fold with DMEM medium into a new 96-well plate and centrifuged at 300 g for 5 min. 100 µl of supernatant from each well was added to the ELISA plate and incubated for 1.5 hours at room temperature. The plate was washed 3 times with PBST, 100 µl of biotinylated rat anti-human IL-6 (working concentration: 1:2000) was added to each well, and placed at 37°C for 1 h. After the plate was washed 3 times with PBST, the residual droplets were removed as much as possible on absorbent paper. Each well was added with 100 µl of Streptavidin HRP and incubated for 1 h at room temperature, the plate was washed 3 times with PBST, 100 µl of TMB was added to each well, and the plate was placed at room temperature (20±5°C) for 5 min away from light. The substrate reaction was terminated by adding 50 µl of 2M H2SO4 termination solution to each well, and the OD values were read at 450 nm using the microplate reader, and the IL-6 expression level of each well was calculated using the software. GraphPad Prism6 was used to analyze the data, graphs were plotted and IC50 was calculated.

The experimental results are shown in Figure 4. The results indicate that the anti-IL-17/VEGF bifunctional fusion proteins 17V05, 17V06, and the positive control 608 monoclonal antibody dose-dependently block the secretion of human IL-17A-induced human IL-6 from HeLa cells, with IC50s of 0.726, 0.778, and 0.877 nM, and that bifunctional fusion proteins and 608 monoclonal antibody show comparable biological activities.

### Embodiment 7. Cellular assay of anti-IL-17/VEGF bifunctional fusion protein blocking the binding of VEGF to receptor KDR

**KDR** cells (purchased from promega, Cat.# GA1082) grown in log phase in adherent culture at a density of approximately 80%-90% were taken and the growth medium was discarded. After one wash with DPBS, the cells were digested with Accutase^{®} solution (Sigma, Cat.# A6964). After neutralizing trypsin, the mixture was centrifuged at 200 g for 5 min, and the cells were resuspended in DMEM medium containing 10% FBS (Gibco Cat.# 11995), counted in Trypan blue, and adjusted to a cell density of 40,000 cells/well, 50 µl/well, and placed at 37°C, 5% CO2. VEGF was diluted to 30 ng/ml with DMEM medium containing 10% FBS, and the antibody was diluted with VEGF-containing medium using a serial dilution method, with 3-fold dilution, 10 gradients. Diluted antibody was added to cell wells at 25 µl per well (final VEGF concentration of 10 ng/ml, antibody initial concentration of 100 nM) and incubated at 37°C for 6 h. After incubation, 75 µl of the detection reagent Bio-Glo (purchased from promega, Cat.# G7940) was added to each well. After 10 minutes of incubation at room temperature, the luminescence was read with a SpectraMax i3x. All data were from duplicate wells, and the resulting signal values were averaged and then fitted by the 4-parameter method, and the data were analyzed using GraphPad Prism6.

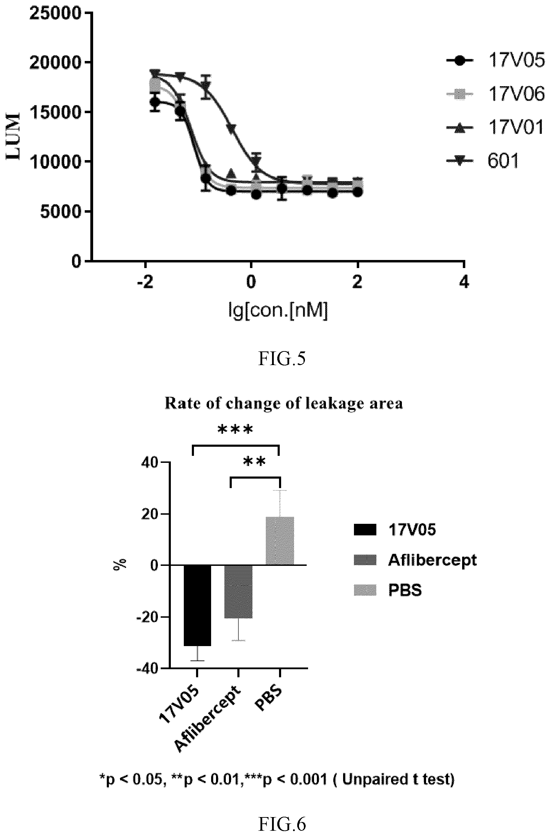

The experimental results are shown in Figure 5: both anti-IL-17/VEGF bifunctional fusion proteins and the positive control Bevacizumab were able to effectively block the interaction between VEGF and its receptor KDR, and the bifunctional fusion proteins had a superior blocking ability. The IC50 for 17V01, 17V05, 17V06 and Bevacizumab were 0.072, 0.084, 0.072 and 0.430 nM, respectively.

### Embodiment 8. Physical stability of anti IL-17/VEGF bifunctional fusion protein

The thermal stability of anti-IL-17/VEGF bifunctional fusion proteins in PBS buffer system was examined using DSC (Differential scanning calorimetry). The samples were displaced into PBS buffer, controlled at 1 mg/ml, and assayed using MicroCal*Vp-Capillary DSC (Malvern). Prior to the assay, the samples and blank buffer were filtered through a 0.22 µm filter membrane. Sample plates were added with 400 µl of sample or blank buffer (6 sets of blank buffer pairs) per well, and the last three pairs of well plates were added with ddH2O for washing. After the samples were added to the sample plates, the sample plates were covered with plastic soft lids. The scanning temperature started at 25°C and ended at 100°C, with a scanning rate of 150°C/h. The specific results are shown in Table 4, where samples 17V01, 17V05 as well as 17V06 proteins showed good thermal stability.

**Table 4: Thermal stability assay data of anti-IL-17/VEGF bifunctional fusion protein**

| Sample | TmOnse (°C) | Tm1 (°C) |
|---|---|---|
| 17V01 | 66.16 | 79.54 |
| 17V05 | 66.55 | 79.58 |
| 17V06 | 65.83 | 79.52 |

### Embodiment 9. Accelerated thermal stability study of anti IL-17/VEGF bifunctional fusion protein

Based on previous studies, it was found that the natural D2 structural domain sequence used in bifunctional fusion proteins showed a small number of heavy chain breaks in accelerated thermal stability studies of certain formulation formulations. The accelerated thermal stability of the modified molecule in the formulated formulation was examined by mutating methionine at position 138.

The 17V01, 17V05, 17V06, and 17V07 proteins were concentrated by ultrafiltration into PBS buffer or histidine buffer (L-histidine, 0.34 mg/ml, respectively; Histidine hydrochloride monohydrate, 1.64 mg/ml; Arginine hydrochloride, 5 mg/ml; α,α-Alginose dihydrate, 50.0 mg/ml; Polysorbate 80, 0.4 mg/ml; pH 5.5±0.5) with protein concentrations between 5-10 mg/mL, incubated at 37°C for 4 weeks, and then sampled for desalination by centrifugal ultrafiltration. 100 µg of protein after desalting by ultrafiltration was taken, added with 1 µl of NEB PNGase F (purchased from NEB, Cat.# P0704L) with a concentration of 500,000 U/ml, incubated at 37°C for 2 h, added with 1 M DTT (purchased from Sangon Biotech (Shanghai) Co., Ltd., Cat.# A620058) until the final concentration is 50 mM, and incubated at 37°C for 30 min. Then, the sample was diluted until the protein concentration is 1.5 mg/ml with 50 mM NH4HCO3, mixed well and centrifuged for 10 min (≥12000 rpm). After separation on an ACQUITY UPLC BEH300 C4 1.7 µm 2.1□50 mm column, the heavy chain molecular weight of the fusion proteins was analyzed by mass spectrometry (Waters UPLC-XEVO G2 Q-TOF Liquid Mass Spectrometry System). The results, as shown in Table 5, show that 17V05, 17V06, and 17V07 molecules have superior accelerated thermal stability compared to 17V01 molecules having the natural D2 structural domain sequence.

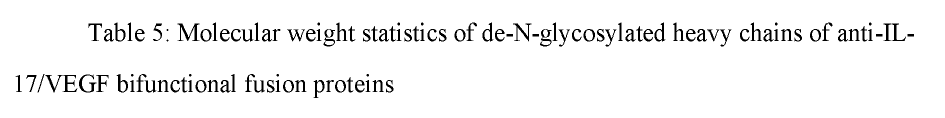

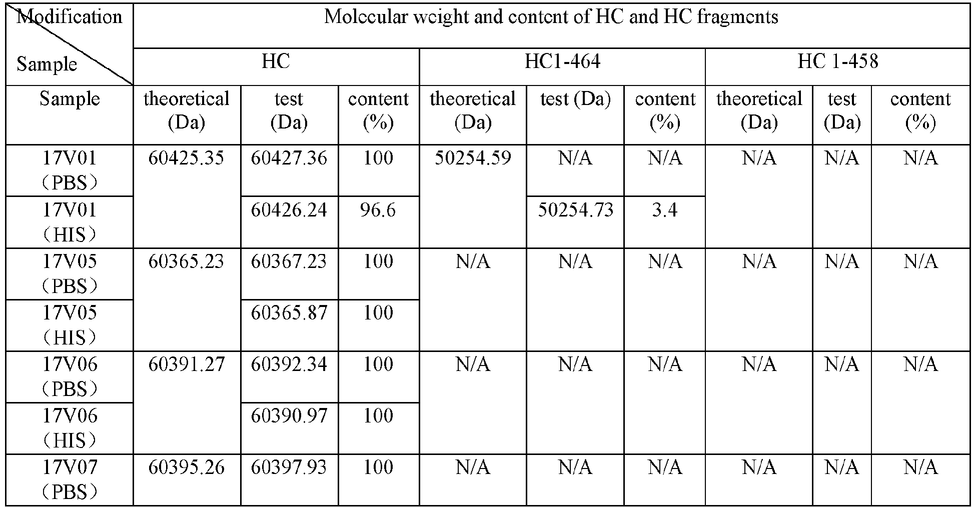

### Embodiment 10. Role of Anti-IL-17/VEGF bifunctional fusion protein in Inhibiting Laser-Induced Choroidal Neovascularization in a Mouse.

Laser irradiation was used to induce choroidal neovascularization and leakage in the mouse fundus choroid to verify the inhibitory effect of IL-17/VEGF bifunctional fusion protein on neovascularization and leakage.

11-12-week-old female humanized IL-17 mice (purchased from Biocytogen) were anesthetized with Zoletil (25-50 mg/kg, i.p.) + xylazine hydrochloride injection (5 mg/kg, i.p.), and a 532 nm laser was used to photocoagulate 3 points equidistant from each eye around the optic papillae at 1.5-2 PD from the optic disc, and to cauterize 3 laser spots. OCT detection was performed immediately after laser modeling to confirm whether the Bruch's membrane was punctured and to exclude light spots that did not puncture the Bruch's membrane or caused retinal hemorrhage. On day 6 (day6) after laser modeling, FFA assay was performed and grouped according to the resulting leakage spot, and 2 µL of 17V05 (17.49 mg/mL) was injected in the anti-IL-17/VEGF bifunctional fusion protein administration group, 2 µL of Aflibercept (10 mg/mL) was injected in the Aflibercept administration group, and 2 µL of PBS was injected in the control group (single administration of bilateral intravitreal injection). After 7 days post the administration (day 14), the FFA assay was performed again, and in vivo CNV leakage was observed using a Heidelberg Spectralis system. Photographs were taken at the early stage of FFA (less than 1 minute) and at the late stage (after 3 minutes), and the area of the fluorescence leakage was counted and graded according to the fluorescence leakage (grades I~IV):
Class I no strong fluorescence;
Grade II Early or mid-stage lesions are hyperfluorescent with no leakage;
Grade III The lesion is hyperfluorescent in the early stages and leaks fluorescence in the late stages;
Grade IV The lesion is brightly hyperfluorescent in the early stages, and leaks fluorescence and extends beyond the boundaries of the burned area in the later stages.

After the examination was completed, the animals inhaled excess carbon dioxide and were euthanized by cervical dislocation, the eyeballs of both eyes of the experimental animals were collected and fixed with 4% PFA at room temperature for 2 hours, after replacement of pre-cooled PBS, the eyeballs were dissected and clipped to remove the anterior segmental tissues, lens, vitreous, and retinas, and the RPE/ Choroid-containing paired slices were left to be stained for immunofluorescence, and the indexes of isolectin B4 and DAPI were detected, and the lesions were photographed at the same magnification and same fluorescence setting. The CNV area was measured by imageJ and statistically analyzed.

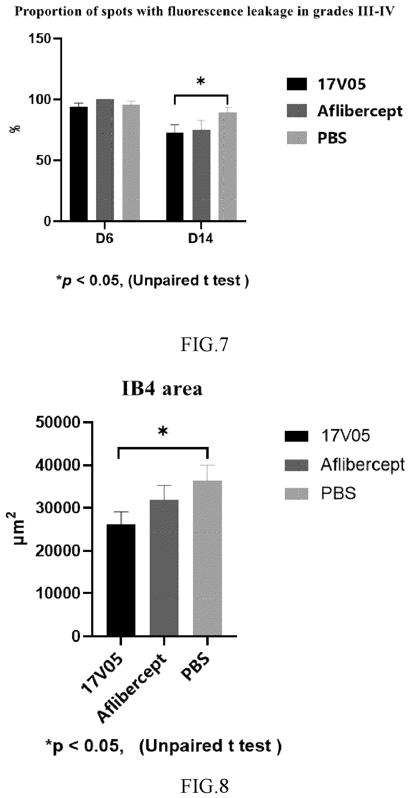

Figure 6 shows the rate of change of vascular leakage area after 7 days post the administration compared with that before administration. At equimolar administration concentrations, both IL-17/VEGF bifunctional fusion proteins and Aflibercept effectively reduced the leakage area, and IL-17/VEGF bifunctional fusion proteins were more efficacious. Figure 7 shows the statistical data of grade 3-4 leaky light spots. After 7 days post the administration (day 14), the number of grade 3-4 leaky light spots in the experimental group given IL-17/VEGF bifunctional fusion proteins was significantly lower compared with the negative control group, and the effect was superior to that of the positive control, Aflibercept. Figure 8 shows the area of neovascularization in the mouse fundus after 7 days post the administration by IB4 immunofluorescence staining after choroidal flat mount, and the area of neovascularization in the experimental group given IL-17/VEGF bifunctional fusion proteins was significantly reduced compared with the negative control group, and the effect was superior to that of the positive control, Aflibercept.

Taken together, the experimental results showed that IL-17/VEGF bifunctional fusion protein could effectively inhibit choroidal neovascularization and leakage in the mouse fundus choroid, and the effect was superior to that of the positive control, Aflibercept.

The above embodiments are intended to illustrate the disclosed embodiments of the present disclosure and are not understood as restrictions on the present disclosure. In addition, various modifications of the present disclosure, as well as variations of the methods of the disclosure, will be apparent to those skilled in the art without departing from the scope of the present disclosure. While the disclosure has been described in detail in connection with various specific preferred embodiments thereof, however, it should be understood that the present disclosure should not be limited to these specific embodiments. In fact, various modifications to the present disclosure as apparent to those skilled in the art are intended to be included within the scope of the present disclosure.

## Claims

1. An anti-IL-17/VEGF bifunctional fusion protein, comprising an anti-IL-17 antibody and a protein that binds specifically to VEGF, the protein that binds specifically to VEGF is VEGFR, the protein that binds specifically to VEGF is linked to the anti-IL-17 antibody either directly or via a linker.

2. The anti-IL-17/VEGF bifunctional fusion protein according to claim 1, **characterized in that** the protein that binds specifically to VEGF is an extracellular domain of VEGFR; preferably, the protein that binds specifically to VEGF is an extracellular domain of VEGFR1.

3. The anti-IL-17/VEGF bifunctional fusion protein according to claim 2, **characterized in that** the protein that binds specifically to VEGF is a D2 domain of VEGFR1.

4. The anti-IL-17/VEGF bifunctional fusion protein according to claim 1, **characterized in that** the anti-IL-17 antibody heavy chain comprises complementary determining regions HCDR1-3, wherein an amino acid sequence of HCDR1 is shown as SEQ ID NO: 14, an amino acid sequence of HCDR2 is shown as SEQ ID NO: 15, and an amino acid sequence of HCDR3 is shown as SEQ ID NO: 16; the anti-IL-17 antibody light chain comprises complementary determining regions LCDR1-3, wherein an amino acid sequence of LCDR1 is shown as SEQ ID NO: 17, an amino acid sequence of LCDR2 is shown as SEQ ID NO: 18, and an amino acid sequence of LCDR3 is shown as SEQ ID NO: 19.

5. The anti-IL-17/VEGF bifunctional fusion protein according to claim 1, **characterized in that** an amino acid sequence of a heavy chain variable region of the anti-IL-17 antibody is shown as SEQ ID NO: 20, and an amino acid sequence of a light chain variable region of the anti-IL-17 antibody is shown as SEQ ID NO: 21.

6. The anti-IL-17/VEGF bifunctional fusion protein according to claim 1, **characterized in that** a heavy chain of the anti-IL-17 antibody has an amino acid sequence shown as SEQ ID NO: 4 and a light chain of the anti-IL-17 antibody has an amino acid sequence shown as SEQ ID NO: 1.

7. The anti-IL-17/VEGF bifunctional fusion protein according to claim 3, **characterized in that** the D2 domain of VEGFR1 is wild-type or mutant, and an amino acid sequence of the D2 domain of wild-type VEGFR1 is shown as SEQ ID NO: 34.

8. The anti-IL-17/VEGF bifunctional fusion protein according to claim 7, **characterized in that** the mutant of the D2 domain of VEGFR1 has a mutation at position 138 of the amino acid sequence shown as SEQ ID NO: 34.

9. The anti-IL-17/VEGF bifunctional fusion protein according to claim 7, **characterized in that** the mutant of the D2 domain of VEGFR1 has a substitution mutation at position 138 of the amino acid sequence shown as SEQ ID NO: 34.

10. The anti-IL-17/VEGF bifunctional fusion protein according to claim 7, **characterized in that** the mutant of the D2 domain of VEGFR1 is selected from 138M/A, 138M/P, and 138M/T.

11. The anti-IL-17/VEGF bifunctional fusion protein according to claim 3, **characterized in that** the C-terminal or N-terminal end of a heavy chain of the anti-IL-17 antibody is connected to the D2 domain of VEGFR1, or, the C-terminal or N-terminal end of a light chain of the anti-IL-17 antibody is connected to the D2 domain of VEGFR1.

12. The anti-IL-17/VEGF bifunctional fusion protein according to claim 1, **characterized in that** a heavy chain of the bifunctional fusion protein has an amino acid sequence shown as SEQ ID NO: 2, SEQ ID NO: 3, SEQ ID NO: 4, SEQ ID NO: 7, SEQ ID NO: 8, SEQ ID NO: 9, or SEQ ID NO: 10; a light chain of the bifunctional fusion protein has an amino acid sequence shown as SEQ ID NO: 1, SEQ ID NO: 5, or SEQ ID NO: 6.

13. A nucleic acid molecule, encoding the anti IL-17/VEGF bifunctional fusion protein according to any one of claims 1-12.

14. The nucleic acid molecule according to claim 13, **characterized in that** a nucleic acid sequence of the nucleic acid molecule encoding a heavy chain of the anti-IL-17/VEGF bifunctional fusion protein is shown as SEQ ID NO: 25, SEQ ID NO: 26, SEQ ID NO: 27, SEQ ID NO: 30, SEQ ID NO: 31, SEQ ID NO: 32, or SEQ ID NO: 33, and a nucleic acid sequence of the nucleic acid molecule encoding a light chain of the anti-IL-17/VEGF bifunctional fusion protein is shown as SEQ ID NO: 24, SEQ ID NO: 28, or SEQ ID NO: 29.

15. An expression vector, comprising the nucleic acid molecule according to claim 13 or 14.

16. A host cell, comprising the expression vector according to claim 15.

17. A method for preparing the anti IL-17/VEGF bifunctional fusion protein according to any one of claims 1-12, comprising the following steps:
a) culturing the host cell according to claim 16 under expression conditions for expression of the anti-IL-17/VEGF bifunctional fusion protein;
b) isolating and purifying the fusion protein in step a).

18. A pharmaceutical composition, comprising an effective amount of the fusion protein according to any one of claims 1-12 and one or more pharmaceutically acceptable carriers or excipients.

19. A use of the anti IL-17/VEGF bifunctional fusion protein according to any one of claims 1-12 and the pharmaceutical composition according to claim 18 in the preparation of drugs for treating diseases associated with IL-17 and/or VEGF overexpression.

20. A use of the anti IL-17/VEGF bifunctional fusion protein according to any one of claims 1-12 and the pharmaceutical composition according to claim 18 in the preparation of drugs for the treatment of inflammatory skin diseases or ophthalmic diseases, wherein the inflammatory skin disease is psoriasis; and/or, the ophthalmic disease is selected from diabetic retinopathy, choroidal neovascularization disease, and macular disease.

21. A method for treating a disease in which IL-17 and/or VEGF is overexpressed, comprising administering to a subject in need thereof the bifunctional fusion protein of any one of claims 1-12 or the pharmaceutical composition of claim 18.

22. The method according to claim 21, **characterized in that** the disease in which IL-17 and/or VEGF is overexpressed is inflammatory skin disease or ophthalmic disease, wherein the inflammatory skin disease is psoriasis; and/or, the ophthalmic disease is selected from diabetic retinopathy, choroidal neovascularization disease, and macular disease.
